# EUROPEAN PATENT APPLICATION

(11) **EP 4 613 744 A1**
(43) Date of publication of application: **10.09.2025**
(21) Application number: 23884351.0
(22) Date of filing: 04.08.2023
(51) Int. Cl.: C07D 401/14, C07D 403/04, C07D 403/14, C07D 413/14, A61K 31/497, A61K 31/5377, A61P 9/10, A61P 25/28, A61P 21/00

(54) **HETEROCYCLIC COMPOUND, PREPARATION METHOD THEREFOR, AND APPLICATION THEREOF**

(30) Priority: 03.11.2022 CN 202211368240
(71) Applicant: Guangdong Zhongke Medicine Research Ltd., Guangzhou, Guangdong 510663 (CN)
(72) Inventor: WANG, Wei, Guangzhou, Guangdong 510663 (CN); LU, Yongzhang, Guangzhou, Guangdong 510663 (CN); TAN, Jinhui, Guangzhou, Guangdong 510663 (CN)
(74) Representative: Bals & Vogel Patentanwälte PartGmbB
(86) International application number: PCT/CN2023/111120
(87) International publication number: WO 2024/093412

(57) **Abstract**

Provided are a heterocyclic compound, a preparation method therefor and use thereof. The heterocyclic compound has a structural formula represented by formula I. The compound has excellent bioavailability, can improve a cerebral blood flow level and reduce a cerebral infarction area in an ischemia-reperfusion animal model, and can be used for treating diseases such as cerebral stroke.

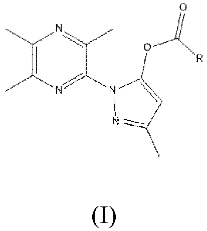

## Description

### TECHNICAL FIELD

The present invention belongs to the field of medicine, and specifically relates to a heterocyclic compound, a preparation method therefor and use thereof.

### BACKGROUND

"Cerebral stroke" is also known as "stroke" or "cerebrovascular accident" (CVA). It is an acute cerebrovascular disease, which involves a group of diseases that cause damage to brain tissues when blood cannot flow into the brain due to sudden rupture of cerebral blood vessels or blockage of the blood vessels, including ischemic stroke and hemorrhagic stroke. The ischemic stroke has a higher incidence than the hemorrhagic stroke, accounting for 60%-70% of a total number of the cerebral stroke. Occlusion and stenosis of the internal carotid artery and the vertebral artery may cause the ischemic stroke in persons with the age of more than 40 years old, including more males than females, and death may be caused in serious situations. The mortality of the hemorrhagic stroke is higher. Surveys show that the cerebral stroke has become a first cause of death in China according to calculation in urban and rural areas and is also a leading cause of disability for Chinese adults. The cerebral stroke has characteristics of a high incidence, high mortality and a high disability rate. Different types of the cerebral stroke have different treatment methods.

Currently, edaravone is mainly used for treating the ischemic stroke. However, because a free radical scavenging effect and an antioxidant effect of the edaravone have been confirmed, various experimental studies also show that the edaravone has a protective effect on various other organ injuries, suggesting that the edaravone not only has an effect limited to treating the ischemic stroke, but also can play a role in treatment of various diseases closely related to oxidative stress. However, due to lower bioavailability, the edaravone can only be used for treating the cerebral stroke by injection, and an oral dosage form is more favorable for treatment of the cerebral stroke in a late stage and ALS (amyotrophic lateral sclerosis). Therefore, it is extremely necessary to find a drug that can be taken orally for treatment of the cerebral stroke.

### SUMMARY

An objective of the present invention is to provide a heterocyclic compound and a preparation method therefor.

The heterocyclic compound provided by the present invention is a compound of formula I or a pharmaceutically acceptable salt thereof: 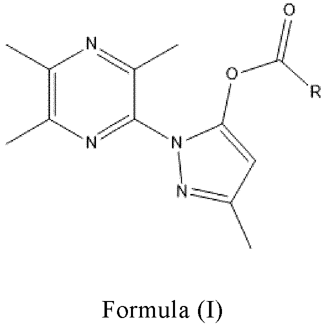
in the Formula (I), R is independently selected from any one of the following groups: (C₁-C₆) alkyl or substituent-containing (C₁-C₆) alkyl, (C₃-C₈) carbocyclicalkyl, (C₂-C₈) alkenyl or substituent-containing (C₂-C₈) alkenyl, (C₂-C₈) alkynyl or substituent-containing (C₂-C₈) alkynyl, (C₆-C₂₀) aryl or substituent-containing (C₆-C₂₀) aryl, (C₂-C₂₀) heterocyclyl or substituent-containing (C₂-C₂₀) heterocyclyl, -ORₐ, -NR_{b}R_{c}, and -SO₂(OR_{d}); and
Rₐ, R_{b}, R_{c} and Rₐ are independently selected from hydrogen, a (C₁-C₅) alkane or a substituent-containing (C₁-C₅) alkane, (C₃-C₈) carbocycliclalkyl, (C₂-C₈) alkenyl or substituent-containing (C₂-C₈) alkenyl, (C₂-C₈) alkynyl or substituent-containing (C₂-C₈) alkynyl, (C₆-C₂₀) aryl or substituent-containing (C₆-C₂₀) aryl, and (C₂-C₂₀) heterocyclyl or substituent-containing (C₂-C₂₀) heterocyclyl.

Further, in the Formula (I), R is independently selected from any one of the following groups: (C₁-C₆) alkyl, (C₁-C₆) alkyl substituted by halogen, a heterocycle containing nitrogen and/or oxygen, a heterocycle containing nitrogen and/or oxygen substituted by a (C₁-C₅) alkane or a substituent-containing (C₁-C₅) alkane, a heterocycle containing nitrogen and/or oxygen substituted by hydroxy or amino, a heterocycle containing nitrogen and/or oxygen substituted by acetyl-substituted hydroxy or amino, a heterocycle containing nitrogen and/or oxygen substituted by methyl- or ethyl-substituted amino, a benzene ring, a benzene ring substituted by (C₁-C₅) alkyl or substituent (halogen)-containing (C₁-C₅) alkyl, a benzene ring substituted by hydroxy or amino, a benzene ring substituted by acetyl-substituted hydroxy or amino, and a benzene ring substituted by methyl- or ethyl-substituted amino.

Preferably, in the Formula (I), R is independently selected from methyl, tert-butyl, trifluoromethyl, phenyl, acetoxyphenyl, pyridyl, pyrrolyl, piperidyl, morpholinyl, and pyrazinyl.

Specifically, the compound of formula I may be any one of the following compounds:

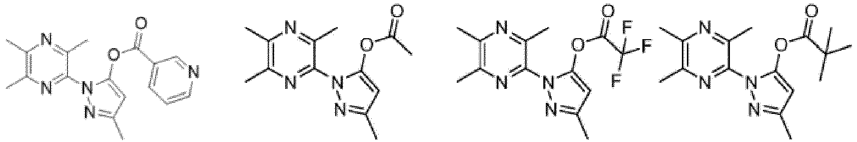

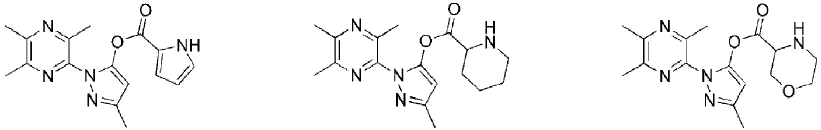

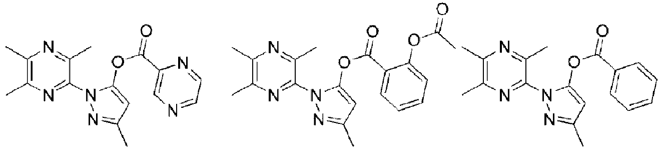

The pharmaceutically acceptable salt of the compound of formula I in the present invention refers to a salt that is suitable for use in contact with tissues of humans and lower animals without occurrence of excessive toxicity, irritation and anaphylaxis within a reliable medical judgment range and is commensurate with a reasonable effect/risk ratio.

It is also found that when R is selected from some commonly used modification groups, many compounds cannot be obtained smoothly. For example, when R is selected from benzyl, phenylethyl, and benzyl and phenylethyl substituted by amino and hydroxy, samples cannot be synthesized smoothly. A formula is shown below:

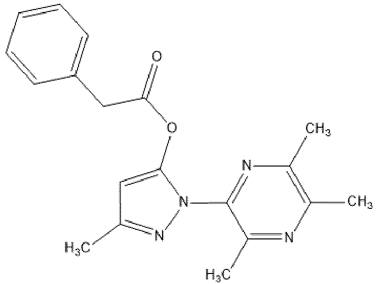

The present invention also provides use of the above compound of formula I or a pharmaceutically acceptable salt thereof in preparation of a drug for prevention and/or treatment of an ischemic brain disease.

The ischemic brain disease includes cerebral stroke, Alzheimer's disease, amyotrophic lateral sclerosis, etc.

The present invention also provides a drug or pharmaceutical composition for prevention and/or treatment of an ischemic brain disease, which includes the above compound of formula I or a pharmaceutically acceptable salt thereof, and a pharmaceutically acceptable carrier.

The drug may be introduced into the body, such as muscles, intradermal tissues, subcutaneous tissues, veins and mucosal tissues by a method of injection, spraying, permeation, absorption, or physical or chemical mediation, or introduced into the body by being mixed or encapsulated with other substances.

Preferably, the ischemic brain disease is a disease related to improvement of brain cells.

Preferably, the ischemic brain disease includes cerebral stroke, Alzheimer's disease, amyotrophic lateral sclerosis, etc.

Preferably, a dosage form of the drug or pharmaceutical composition is an oral solid preparation or a liquid preparation. Preferably, the liquid preparation is an injection. All of the above various dosage forms of drugs can be prepared according to conventional methods in the pharmaceutical field.

Compared with the prior art, the present invention has the following beneficial effects.

The compound of formula I of the present invention can improve a cerebral blood flow and reduce an infarction area in an ischemia-reperfusion animal through a gavage method, suggesting that it can be prepared in the form of an oral solid preparation for treatment of diseases such as cerebral stroke, Alzheimer's disease, and ALS.

### DETAILED DESCRIPTION OF EMBODIMENTS

The present invention is further described in detail below in conjunction with specific embodiments, and examples given below are intended only to illustrate the present invention, rather than to limit the scope of the present invention. The examples provided below can serve as a guide for those of ordinary skill in the technical field to make further improvement, and do not constitute limitations to the present invention in any way.

Experimental methods in the following examples, unless otherwise specified, are all conventional methods, and are used according to technologies or conditions described in documents in the art or instructions of products. Materials, reagents and the like used in the following examples, unless otherwise specified, are all commercially available.

### Example 1, Synthesis of 3-methyl-1-(3,5,6-trimethylpyrazin-2-yl)-1H-pyrazol-5-nicotinate (test drug 1)

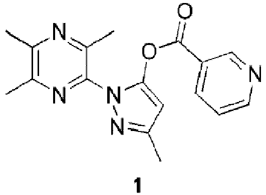

### 1) 2,3,5-trimethylpyrazin-1-oxide

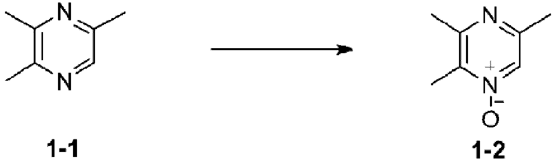

2,3,5-trimethylpyrazine (20.0 g, 0.164 mol) was dissolved in acetic acid (100 mL) and heated to 80°C in oil bath, and sodium perborate tetrahydrate (38.0 g, 0.247 mol) was added in batches and stirred to carry out a reaction at a constant temperature for 20 h. Filtration was performed to remove an insoluble substance in the reaction solution, concentration was performed, and purification was performed by column chromatography to obtain 2,3,5-trimethylpyrazin-1-oxide as a yellow transparent liquid (18.3 g, 80.9%). ¹H NMR (DMSO-d6 400 MHz) δ9.24 (s, 1H), 2.45 (s, 3H), 2.34 (s, 6H). ESI-MS m/z: 139.1 [M+H]⁺.

### 2) 2-chloro-3,5,6-trimethylpyrazine

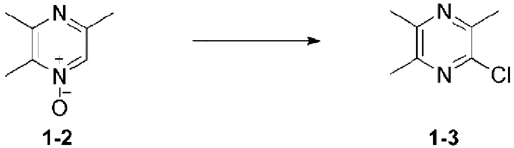

Phosphorus oxychloride (90 mL) and a catalytic amount of concentrated sulfuric acid were added into a three-neck round-bottom flask and cooled to 10°C under an ice water bath, the 2,3,5-trimethylpyrazin-1-oxide (18.0 g, 0.130 mol) was added dropwise, and after the dropping was completed, the mixture was slowly heated to 100°C and stirred at a constant temperature for 20 h. The reaction solution was subjected to reduced pressure distillation. The residual solution was poured into an aqueous solution of saturated sodium bicarbonate, extracted with dichloromethane, concentrated, and purified by column chromatography to obtain 2-chloro-3,5,6-trimethylpyrazine as a white solid (7.5 g, 37.0%). ¹H NMR (DMSO-d6 400 MHz) δ2.56 (s, 3H), 2.47 (s, 6H). ESI-MS m/z: 157.6 [M+H]⁺.

### 3) 2-hydrazinyl-3,5,6-trimethylpyrazine

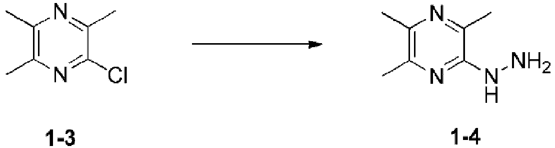

The 2-chloro-3,5,6-trimethylpyrazine (7.0 g, 44.9 mmol) and 80% hydrazine hydrate (100 mL) were mixed and refluxed to carry out a reaction at 100°C for 48 h. The reaction solution was concentrated and purified by column chromatography to obtain 2-hydrazinyl-3,5,6-trimethylpyrazine as a white solid (4.5 g, 65.9%). ¹H NMR (DMSO-d6 400 MHz) δ7.25 (s, 1H), 4.08 (s, 2H), 2.29-2.19 (m, 9H). ESI-MS m/z: 157.6 [M+H]⁺.

### 4) 3-methyl-1-(3,5,6-trimethylpyrazin-2-yl)-1H-pyrazol-5-ol

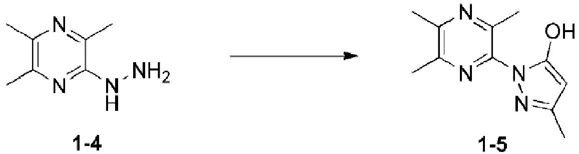

The 2-hydrazinyl-3,5,6-trimethylpyrazine (4.0 g, 26.3 mmol), ethyl acetoacetate (6.8 g, 52.6 mmol) and water (20 mL) were mixed and stirred at a constant temperature of 100°C for 24 h. The reaction solution was concentrated and purified by column chromatography to obtain 3-methyl-1-(3,5,6-trimethylpyrazin-2-yl)-1H-pyrazol-5-ol as a yellow oily substance (2.2 g, 38.4%). ¹H NMR (CDCl₃ 400 MHz) δ3.42 (s, 2H), 2.54-2.21 (m, 12H). ESI-MS m/z: 219.1 [M+H]⁺.

### 5) 3-methyl-1-(3,5,6-trimethylpyrazin-2-yl)-1H-pyrazol-5-nicotinate

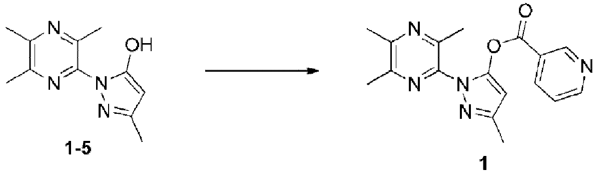

The 3-methyl-1-(3,5,6-trimethylpyrazin-2-yl)-1H-pyrazol-5-ol (0.2 g, 0.92 mmol) and triethylamine (0.19 g, 1.84 mmol) were dissolved in dichloromethane (5 mL), and a dichloromethane (2 mL) solution of nicotinoyl chloride hydrochloride (0.2 g, 1.1 mmol) was added dropwise to carry out a reaction at room temperature for 5 h. The reaction solution was concentrated and purified by column chromatography to obtain 3-methyl-1-(3,5,6-trimethylpyrazin-2-yl)-1H-pyrazol-5-nicotinate as a white solid (0.15 g, 50.5%). ¹H NMR (DMSO-d6 400 MHz) δ9.13-7.41 (m, 4H), 6.30 (s, 1H), 2.55-2.37 (m, 12H). ESI-MS m/z: 324.1 [M+H]⁺.

### Example 2, Synthesis of 3-methyl-1-(3,5,6-trimethylpyrazin-2-yl)-1H-pyrazol-5-acetate (test drug 2)

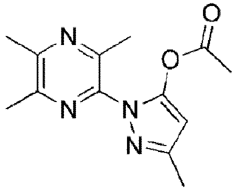

Combining with the synthesis method in Example 1, acetic anhydride as a raw material was allowed to undergo an esterification reaction with the intermediate 1-5 to obtain 3-methyl-1-(3,5,6-trimethylpyrazin-2-yl)-1H-pyrazol-5-acetate as a white solid. ¹H NMR (DMSO-d6 400 MHz) δ6.30 (s, 1H), 2.55-2.37 (m, 15H). ESI-MS m/z: 261.1 [M+H]⁺.

### Example 3, Synthesis of 3-methyl-1-(3,5,6-trimethylpyrazin-2-yl)-1H-pyrazol-5-trifluoroacetate

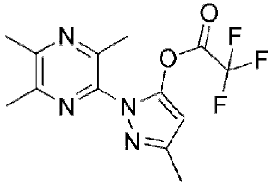

Combining with the synthesis method in Example 1, trifluoroacetic anhydride as a raw material was allowed to undergo an esterification reaction with the intermediate 1-5 to obtain 3-methyl-1-(3,5,6-trimethylpyrazin-2-yl)-1H-pyrazol-5-trifluoroacetate as a white solid. ¹H NMR (DMSO-d6 400 MHz) δ6.30 (s, 1H), 2.55-2.37 (m, 12 H). ESI-MS m/z: 315.1 [M+H]⁺.

### Example 4, Synthesis of 3-methyl-1-(3,5,6-trimethylpyrazin-2-yl)-1H-pyrazol-5-pivalate (test drug 5)

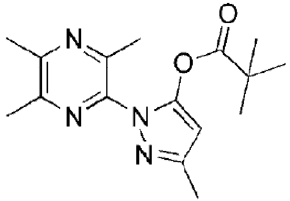

Combining with the synthesis method in Example 1, pivaloyl chloride as a raw material was allowed to undergo an esterification reaction with the intermediate 1-5 to obtain 3-methyl-1-(3,5,6-trimethylpyrazin-2-yl)-1H-pyrazol-5-pivalate as a white solid. ¹H NMR (DMSO-d6 400 MHz) δ6.03 (s, 1H), 2.55-2.37 (m, 12H), 1.25 (s, 9 H). ESI-MS m/z: 303.1 [M+H]⁺.

### Example 5, Synthesis of 3-methyl-1-(3,5,6-trimethylpyrazin-2-yl)-1H-pyrazol-5-yl-1H-pyrrol-2-carbo xylate

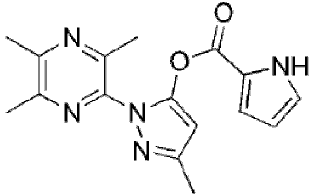

Combining with the synthesis method in Example 1, 1H-pyrrol-2-carboxylic acid as a raw material was allowed to undergo an esterification reaction with the intermediate 1-5 to obtain 3-methyl-1-(3,5,6-trimethylpyrazin-2-yl)-1H-pyrazol-5-yl-1H-pyrrol-2-carboxyla te as a white solid. ¹H NMR (DMSO-d6 400 MHz) δ7.56-6.65 (m, 3H), 6.03 (s, 1H), 2.55-2.37 (m, 12H). ESI-MS m/z: 312.1 [M+H]⁺.

### Example 6, Synthesis of 3-methyl-1-(3,5,6-trimethylpyrazin-2-yl)-1H-pyrazol-5-yl-piperidin-2-carbox ylate

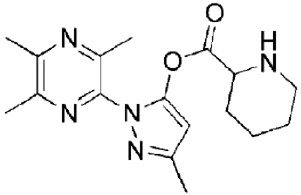

Combining with the synthesis method in Example 1, piperidin-2-carboxylic acid as a raw material was allowed to undergo an esterification reaction with the intermediate 1-5 to obtain 3-methyl-1-(3,5,6-trimethylpyrazin-2-yl)-1H-pyrazol-5-yl-piperidin-2-carboxylat e as a white solid. ¹H NMR (DMSO-d6 400 MHz) δ6.03 (s, 1H), 3.75 (m, 1H), 2.75-2.37 (m, 14 H), 1.62-1.47 (m, 6 H). ESI-MS m/z: 330.1 [M+H]⁺.

### Example 7, Synthesis of 3-methyl-1-(3,5,6-trimethylpyrazin-2-yl)-1H-pyrazol-5-yl-morpholino-2-carb oxylate

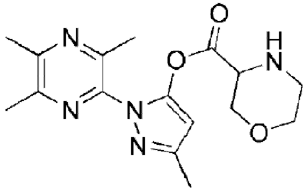

Combining with the synthesis method in Example 1, morpholino-2-carboxylic acid as a raw material was allowed to undergo an esterification reaction with the intermediate 1-5 to obtain 3-methyl-1-(3,5,6-trimethylpyrazin-2-yl)-1H-pyrazol-5-yl-morpholino-2-carboxy late as a white solid. ¹H NMR (DMSO-d6 400 MHz) δ6.33 (s, 1H), 3.75-3.21 (m, 7H), 2.75-2.37 (m, 12H). ESI-MS m/z: 332.1 [M+H]⁺.

### Example 8, Synthesis of 3-methyl-1-(3,5,6-trimethylpyrazin-2-yl)-1H-pyrazol-5-yl-pyrazin-2-carboxy late

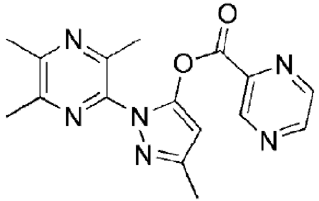

Combining with the synthesis method in Example 1, pyrazin-2-carboxylic acid as a raw material was allowed to undergo an esterification reaction with the intermediate 1-5 to obtain 3-methyl-1-(3,5,6-trimethylpyrazin-2-yl)-1H-pyrazol-5-yl-pyrazin-2-carboxylate as a white solid. ¹H NMR (DMSO-d6 400 MHz) δ9.36-8.78 (m, 3H), 6.03 (s, 1H), 2.75-2.37 (m, 12H). ESI-MS m/z: 325.1 [M+H]⁺.

### Example 9, Synthesis of 3-methyl-1-(3,5,6-trimethylpyrazin-2-yl)-1H-pyrazol-5-yl-2-acetoxybenzoate (test drug 3)

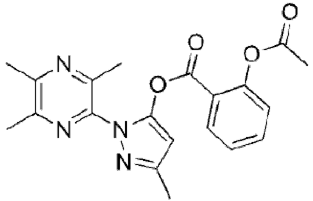

Combining with the synthesis method in Example 1, acetylsalicylic acid as a raw material was allowed to undergo an esterification reaction with the intermediate 1-5 to obtain 3-methyl-1-(3,5,6-trimethylpyrazin-2-yl)-1H-pyrazol-5-yl-2-acetoxybenzoate as a white solid. ¹H NMR (DMSO-d6 400 MHz) δ8.18-7.76 (m, 4H), 6.03 (s, 1H), 2.75-2.37 (m, 15H). ESI-MS m/z: 381.1 [M+H]⁺.

### Example 10, Synthesis of 3-methyl-1-(3,5,6-trimethylpyrazin-2-yl)-1H-pyrazol-5-yl-benzoate (test drug 4)

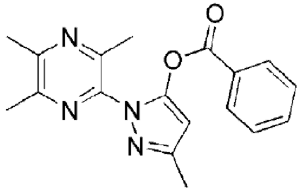

Combining with the synthesis method in Example 1, benzoic acid as a raw material was allowed to undergo an esterification reaction with the intermediate 1-5 to obtain 3-methyl-1-(3,5,6-trimethylpyrazin-2-yl)-1H-pyrazol-5-yl-benzoate as a white solid. ¹H NMR (DMSO-d6 400 MHz) δ8.11-7.66 (m, 5H), 6.03 (s, 1H), 2.75-2.37 (m, 12H). ESI-MS m/z: 323.1 [M+H]⁺.

### Example 11. Drug efficacy test

Rat models with focal cerebral ischemia-reperfusion (tMCAO) were prepared by using a suture-occluded method, and therapeutic effects of the test drugs on the tMCAO model rats were investigated.

A specific modeling method for the rat models with focal cerebral ischemia-reperfusion (tMCAO) is as follows. At 12 h before surgery, the rats were fed at about 30 g/piece. After induced anesthesia with 3-4% isoflurane and maintained anesthesia with 1.5-2.5% isoflurane were performed, the rats were placed at supine positions on an operating table at a constant temperature of 37°C. The skin in middle lines of necks was cut, subcutaneous tissues and muscles were bluntly separated, and common carotid arteries on right sides, internal carotid arteries and external carotid arteries were separated. Two ligation threads were allowed to penetrate through the external carotid arteries to ligate distal ends, an artery clamp was placed at the common carotid arteries and the internal carotid arteries, respectively, small incisions were made at 45° diagonally on the external carotid arteries at about 4 mm away from bifurcations of the common carotid arteries, and suitable suture occlusions were inserted to gently ligate proximal ends. The external carotid arteries were cut, directions and angles of the suture occlusions were adjusted, the suture occlusions were gently pushed and inserted into the internal carotid arteries, and then the artery clamps were released. The suture occlusions were continuously inserted until black dot marks of the suture occlusions reached junctions of the internal carotid arteries and the common carotid arteries at a length of about 18-20 mm. The proximal ends of the external carotid arteries were tightened, the suture occlusions were fixed, the skin was sutured after it was confirmed that there was no bleeding, the body temperature of the rats was maintained, and meloxicam as an analgesic drug was injected subcutaneously at 1 mg/kg.

At 2 h after ischemia, induced anesthesia with 3-4% isoflurane and maintained anesthesia with 1.5-2.5% isoflurane were performed, the skin sutures were cut, the ligation threads at the proximal ends of the external carotid arteries were opened, the suture occlusions were taken out, reperfusion was performed, ligation was performed again, and the skin was sutured after it was confirmed that there was no bleeding. In a sham-operated control group, other steps were the same as above except that the suture occlusions were inserted and pulled out immediately and the reperfusion was not required. After the surgery, the rats were allowed to lie prostrate and return to a cage.

When the animals were awake after modeling, behavioral scoring was performed on the animals with reference to a 4-point scoring standard of Zea Longa. The animals with scores of 1-3 points were judged as successfully modeled animals, and the animals not meeting requirements were eliminated.

Method: SPF-grade male SD rats were used, the various tMCAO rat models were prepared by using the suture-occluded method, and the successfully modeled animals were randomly divided into 6 groups including a model control group, a test drug group 1, a test drug group 2, a test drug group 3, a test drug group 4 and a test drug group 5, with 6 or more animals in each group. In addition, the sham-operated control group was set, which involved 6 animals with the suture occlusions inserted and pulled out immediately in the surgery. At 4 h after the cerebral ischemia (at 4 h after the suture occlusions were pulled out in the sham-operated group), the various groups were given the various drugs or a vehicle for the first time. In each group, an administration dose was 3×10⁻⁵ mol/kg, an administration volume was 3 mL/kg, administration was performed for a total of 3 times, and an administration time interval of every two times was 24 h. The vehicle was injected by catheterization into jugular veins on right sides in the sham-operated control group and the model control group, and the test drugs were injected intravenously in the other groups, wherein the administration time was 20 min. The cerebral blood flow was detected before the surgery, before the first administration and at 24 h after the last administration, and the decrease percentage, recovery percentage and improvement percentage of the cerebral blood flow were calculated. At about 24 h after completion of the last administration, a latent period of falling in a rotarod test, behavioral scores and the percentage of cerebral infarction area were detected.

Results: All the test drug groups are significantly better than the model control group, and the groups 1, 3 and 4 are better than the groups 2 and 5.

Vehicle: The vehicle was obtained by evenly mixing 5 mL of DMSO, 10 mL of Solutol and 85 mL of a sodium chloride injection (0.9%).

Test drugs: The test drugs were weighed, dissolved with the vehicle and diluted to 20 ml.

### 1. Cerebral blood flow

The cerebral blood flow was determined before the surgery, before the first administration and at 24 h after the last administration in each group, respectively. The last detection time was after the behavioral scoring.

### 2. Percentage of cerebral infarction area

After the last cerebral blood flow was detected, the animals in various groups were anesthetized and killed by decapitation, brain tissues were quickly taken out, and brains were separated and frozen at -80°C for about 5 min. The brains were evenly cut into 5 slices, quickly placed in a 1-2% phosphate buffer solution of triphenyltetrazolium chloride (TTC) for incubation in the dark at 37°C until normal tissues were red and cerebral infarction tissues were white according to observation by naked eyes, and then photographed. A white part tissue area (cerebral infarction area) and a total brain area were analyzed using image analysis software ImagePro Plus 6.0. The percentage of cerebral infarction area was calculated according to a formula: percentage of cerebral infarction area (%)=cerebral infarction area/total brain area×100%.

**Table 1 Impacts of drugs on a latent period of falling in a rotarod test of rats (x̅±s)**

| Group | Test drug | Administration route | Administration dose (mol/kg) | Animal number (piece) | Latent period of falling (s) |
|---|---|---|---|---|---|
| Sham-operated control group (A) | Vehicle | Gavage | / | 6 | 49.3±6.1 |
| Model control group (B) | Vehicle | Gavage | / | 7 | 19.4±13.6^{##} |
| Test drug 1 | | Gavage | 3×10⁻⁵ | 7 | 48.7±14.0** |
| Test drug 2 | | Gavage | 3×10⁻⁵ | 6 | 35.2±23.3** |
| Test drug 3 | | Gavage | 3×10⁻⁵ | 7 | 49.7±12.3** |
| Test drug 4 | | Gavage | 3×10⁻⁵ | 6 | 43.8±14.9** |
| Test drug 5 | | Gavage | 3×10⁻⁵ | 6 | 31.5±14.5^ |

| | | | | | |
|---|---|---|---|---|---|
| Note: vs the sham-operated control group: ##p<0.01; vs the model control group: **p<0.01; and vs the high-dose test drug group l: ^, p<0.05. | | | | | |

### 3. Behavioral scores

As can be seen from Table 2, compared with the sham-operated control group, the behavioral scores of the rats in the model control group are significantly increased (p<0.01); and compared with the model control group, the behavioral scores of the rats in the test drug groups are significantly decreased (p<0.05 or 0.01).

**Table 2. Impacts of drugs on behavioral scores of rats (x̅+s)**

| Group | Test drug | Administration route | Administration dose (mol/kg) | Animal number (piece) | Behavioral score |
|---|---|---|---|---|---|
| Sham-operated control group (A) | Vehicle | Gavage | / | 6 | 0.0±0.0 |
| Model control group (B) | Vehicle | Gavage | / | 7 | 3.1±0.8^{##} |
| Test drug 1 | | Intravenous injection | 3×10⁻⁵ | 7 | 1.9±0.6* |
| | | Gavage | | | |
| Test drug 2 | | Gavage | 3×10⁻⁵ | 6 | 2.3±0.7 |
| Test drug 3 | | Gavage | 6×10⁻⁵ | 7 | 1.6±0.7** |
| Test drug 4 | | Gavage | 3×10⁻⁵ | 6 | 1.7±0.7* |
| Test drug 5 | | Gavage | 6×10⁻⁵ | 6 | 2.7±0.9 |

| | | | | | |
|---|---|---|---|---|---|
| Note: vs the sham-operated control group: ##p<0.01; and vs the model control group: *p<0.05, ** p<0.01. | | | | | |

### 4. Cerebral blood flow

**Table 3. Impacts of drugs on the cerebral blood flow of rats (x̅±s)**

| Group | Animal number (piece) | Cerebral blood flow before ischemia (BPU) | Before administration | | 24 h after last administration | | Cerebral blood flow improvement percentage (%) |
|---|---|---|---|---|---|---|---|
| | | | Cerebral blood flow (BPU) | Decrease percentage (%) | Cerebral blood flow (BPU) | Recovery percentage (%) | |
| Sham-opera ted control group (A) | 6 | 349.2±35.6 | 324.4±25.7 | 6.9±3.1 | 339.4±29.0 | 97.5±5.8 | / |
| Model control group (B) | 7 | 336.3±18.6 | 196.3±12.5 ^{##} | 41.5±3.8^{##} | 219.2±20.5^{##} | 65.4±6.5^{##} | 6.9±8.4 |
| Test drug 1 | 7 | 347.5±19.3 | 204.6±10.3 | 40.9±5.2 | 304.8±12.7** | 87.8±2.0** | 28.7±4.9** |
| Test drug 2 | 6 | 371.8±51.6 | 213.3±16.6 | 41.6±8.6 | 367.8±36.9* | 79.3±8.9* | 20.9±13.8* |
| Test drug 3 | 7 | 356.5±56.6 | 210.3±15.1 | 40.1±6.3 | 321.4±54.0** | 90.1±4.2**^{&&} | 30.3±7.1** |
| Test drug 4 | 6 | 370.2±17.2 | 220.8±16.4 * | 40.0±7.1 | 321.2±17.0** | 87.0±5.8**^{&} | 27.0±7.3** |
| Test drug 5 | 6 | 337.9±26.0 | 203.3±8.1 | 39.5±4.4 | 275.0±20.5* | 81.6±5.9**^ | 21.2±4.7*^ |

| | | | | | | | |
|---|---|---|---|---|---|---|---|
| Note: vs the sham-operated control group: ##p<0.01; vs the model control group: *p<0.05, **p<0.01; vs the high-dose test drug group 1: ^p<0.05; and vs the low-dose test drug group 1: &p<0.05, &&p<0.01. | | | | | | | |

### 5. Percentage of cerebral infarction area

**Table 4. Impacts of drugs on the percentage of cerebral infarction area of rats**

| (*x̅*±s) | | | | | |
|---|---|---|---|---|---|
| Group | Test drug | Administration route | Administration dose (mol/kg) | Animal number (piece) | Percentage of cerebral infarction |
| | | | | | area (%) |
| Sham-operated control group (A) | Vehicle | Gavage | / | 6 | 0.4±0.2 |
| Model control group (B) | Vehicle | Gavage | / | 7 | 9.9±3.5^{##} |
| Test drug 1 | | Gavage | 3×10⁻⁵ | 7 | 4.6±1.2** |
| Test drug 2 | | Gavage | 3×10⁻⁵ | 6 | 5.8±2.2* |
| Test drug 3 | | Gavage | 6×10⁻⁵ | 7 | 3.5±1.6**^{&} |
| Test drug 4 | | Gavage | 3×10⁻⁵ | 6 | 4.5±2.5** |
| Test drug 5 | | Gavage | 6×10⁻⁵ | 6 | 2.9±1.0** |

| | | | | | |
|---|---|---|---|---|---|
| Note: vs the sham-operated control group: ## p<0.01; vs the model control group: *p<0.05, **p<0.01; and vs the low-dose test drug group 1: &p<0.05. | | | | | |

All of the test drug groups can significantly improve the latent period of falling, the behavioral scores, the percentage of cerebral infarction area, the cerebral blood flow, the recovery percentage and the improvement percentage of the model animals. The test drug groups 1, 3 and 4 are better than the groups 2 and 5.

## Claims

1. A compound of formula I or a pharmaceutically acceptable salt thereof: 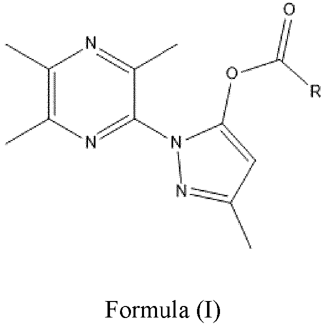
wherein in the Formula (I), R is independently selected from any one of the following groups: (C₁-C₆) alkyl or substituent-containing (C₁-C₆) alkyl, (C₃-C₈) carbocyclicalkyl, (C₂-C₈) alkenyl or substituent-containing (C₂-C₈) alkenyl, (C₂-C₈) alkynyl or substituent-containing (C₂-C₈) alkynyl, (C₆-C₂₀) aryl or substituent-containing (C₆-C₂₀) aryl, (C₂-C₂₀) heterocyclyl or substituent-containing (C₂-C₂₀) heterocyclyl, -ORₐ, -NR_{b}R_{c}, and -SO₂(OR_{d}); and
Rₐ, R_{b}, R_{c} and R_{d} are independently selected from hydrogen, a (C₁-C₅) alkane or a substituent-containing (C₁-C₅) alkane, (C₃-C₈) carbocyclicalkyl, (C₂-C₈) alkenyl or substituent-containing (C₂-C₈) alkenyl, (C₂-C₈) alkynyl or substituent-containing (C₂-C₈) alkynyl, (C₆-C₂₀) aryl or substituent-containing (C₆-C₂₀) aryl, and (C₂-C₂₀) heterocyclyl or substituent-containing (C₂-C₂₀) heterocyclyl.

2. The compound or a pharmaceutically acceptable salt thereof according to claim 1, wherein
in the Formula (I), R is independently selected from any one of the following groups: (C₁-C₆) alkyl, (C₁-C₆) alkyl substituted by halogen, a heterocycle containing nitrogen and/or oxygen, a heterocycle containing nitrogen and/or oxygen substituted by a (C₁-C₅) alkane or a substituent-containing (C₁-C₅) alkane, a heterocycle containing nitrogen and/or oxygen substituted by hydroxy or amino, a heterocycle containing nitrogen and/or oxygen substituted by acetyl-substituted hydroxy or amino, a heterocycle containing nitrogen and/or oxygen substituted by methyl- or ethyl-substituted amino, a benzene ring, a benzene ring substituted by (C₁-C₅) alkyl or substituent (halogen)-containing (C₁-C₅) alkyl, a benzene ring substituted by hydroxy or amino, a benzene ring substituted by acetyl-substituted hydroxy or amino, and a benzene ring substituted by methyl- or ethyl-substituted amino.

3. The compound or a pharmaceutically acceptable salt thereof according to claim 2, wherein
in the Formula (I), R is independently selected from methyl, tert-butyl, trifluoromethyl, phenyl, acetoxyphenyl, pyridyl, pyrrolyl, piperidyl, morpholinyl, and pyrazinyl.

4. The compound or a pharmaceutically acceptable salt thereof according to claim 3, wherein
the compound of formula I is any one of the following compounds:

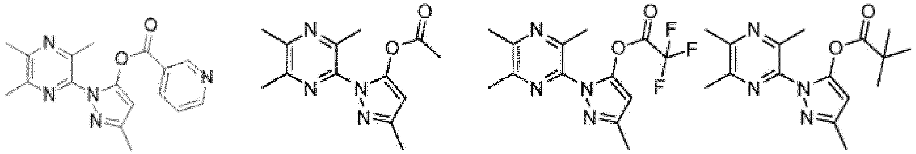

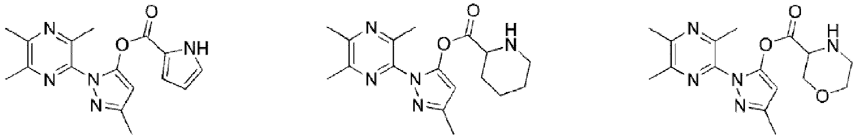

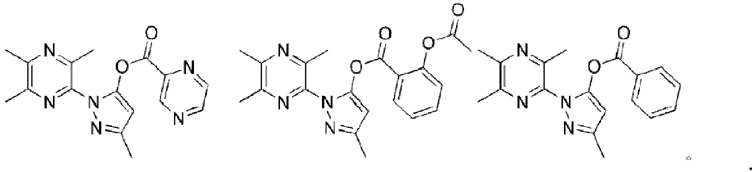

5. Use of the compound or a pharmaceutically acceptable salt thereof according to any one of claims 1-4 in preparation of a drug for prevention and/or treatment of an ischemic brain disease.

6. The use according to claim 5, wherein the ischemic brain disease comprises cerebral stroke, Alzheimer's disease, and amyotrophic lateral sclerosis.

7. A drug or pharmaceutical composition for prevention and/or treatment of an ischemic brain disease, comprising the compound or a pharmaceutically acceptable salt thereof according to any one of claims 1-4, and a pharmaceutically acceptable carrier.

8. The drug or pharmaceutical composition according to claim 7, wherein the disease is a disease related to improvement of brain cells.

9. The drug or pharmaceutical composition according to claim 7, wherein the ischemic brain disease comprises cerebral stroke, Alzheimer's disease, and amyotrophic lateral sclerosis.

10. The drug or pharmaceutical composition according to any one of claims 7-9, wherein a dosage form of the drug or pharmaceutical composition is an oral solid preparation or a liquid preparation.
